# EUROPÄISCHE PATENTSCHRIFT

(11) **EP 2 249 952 B1**
(45) Veröffentlichungstag und Bekanntmachung des Hinweises auf die Patenterteilung: **09.09.2020**
(21) Anmeldenummer: 09718821.3
(22) Anmeldetag: 13.03.2009
(51) Int. Cl.: B01J 2/16, A61K 9/20, A61K 47/38

(54) **VERFAHREN ZUR HERSTELLEN EINES TABLETTIERHILFSMITTELS SOWIE TABLETTIERHILFSMITTEL**
PROCESS FOR PRODUCING A TABLETTING AID AND A TABLETTING AID
PROCÉDÉ DE FABRICATION D'UN ADJUVANT POUR COMPRIMÉS ET ADJUVANT POUR COMPRIMÉS

(30) Priorität: 14.03.2008 DE 102008014237
(43) Veröffentlichungstag der Anmeldung: 17.11.2010
(73) Patentinhaber: J. Rettenmaier & Söhne GmbH + Co. KG, 73494 Rosenberg (DE)
(72) Erfinder: STOYANOV, Edmont, 73433 Aalen (DE); VOLLMER, Reinhard, 55452 Windesheim (DE); GÖTZ, Tobias, 73494 Rosenberg (DE)
(74) Vertreter: Dr. Weitzel & Partner
(86) Internationale Anmeldenummer: PCT/EP2009/001872
(87) Internationale Veröffentlichungsnummer: WO 2009/112287

(56) Entgegenhaltungen:
- EP-A- 0 192 173
- EP-A- 0 819 429
- WO-A-00/13671
- DE-C2- 3 690 101
- GB-A- 1 211 361
- US-A- 4 237 814
- US-A1- 2003 147 957
- US-A1- 2005 008 706
- US-A1- 2007 137 561
- US-A1- 2007 137 561

## Beschreibung

Die Erfindung betrifft ein Verfahren zur Herstellung eines Tablettierhilfsmittels sowie ein mittels dieses Verfahrens hergestelltes Tablettierhilfsmittel.

Direktverpressung ist die meist benutzte Methode für die Tablettenherstellung. Das Verfahren besteht aus verschiedenen Mischschritten. Zuerst werden der Wirkstoff und alle Hilfsstoffe (hauptsächlich Füller, Binder und Sprengmittel) außer dem Schmiermittel miteinander vermischt. Dies kann in einem einzigen Mischschritt vorgenommen werden, wobei Füller, Binder, Sprengmittel gemeinsam dem Wirkstoff zugegeben werden. Stattdessen können auch mehrere aufeinanderfolgende Mischschritte vorgenommen werden. Sodann wird das Schmiermittel dem Gemisch zugegeben und wiederum mit diesem gemischt. Das Ergebnis ist eine Tablettiermischung. Diese wird auf einer Tablettenpresse verpresst.

In der pharmazeutischen Technologie gibt es kombinierte Hilfsstoffe (Direkttablettierhilfsstoffe). Das sind kombinierte Tablettierhilfsstoffe, die aus mehreren Einzelstoffen (sehr häufig Füller, Binder und Sprengmittel) durch Coprocessing (zum Beispiel Sprühtrocknung, Kompaktierung oder Granulierung) hergestellt worden sind. Diese multifunktionellen Materialien zeigen gewisse Vorteile gegenüber der physikalischen Mischung aus den einzelnen Komponenten. Siehe EP 0 819 429. Ein weiteres Konzept ist in der US 2005/008706 A1 beschrieben.

Die meisten bekannten Kombinationen aus Füller, Binder, Sprengmittel, enthalten kein Schmiermittel. Dafür gibt es verschiedene Gründe, vor allem Overmixing-Gefahr, niedrigere Tablettenhärte und Deckeln der Tabletten.

Der Erfindung liegt die Aufgabe zugrunde, ein Verfahren und ein

### BESTÄTIGUNGSKOPIE

Tablettierhilfsmittel anzugeben, um vor allem die folgenden Vorteile zu erzielen:
- niedrige Ausstoßkräfte nach dem Verpressen der Tablettiermischung,
- gute Schmiereigenschaften und gute Fließeigenschaften der Tablettiermischung,
- ausreichende Tablettenhärte,
- geringer Abrieb der Tablette,
- geringe Feuchtigkeitsempfindlichkeit.

Diese Aufgabe wird durch die Merkmale der selbständigen Ansprüche gelöst. Der erfindungsgemäße Tablettierhilfsstoff enthält mehrere Einzelstoffe, im Allgemeinen einen Füller, einen Binder, ein Sprengmittel sowie ein Schmiermittel. Sämtliche der genannten Stoffe liegen somit in einem einzigen Stoffgemisch vor; dieses kann somit als multifunktional bezeichnet werden. Dies hat den großen Vorteil, dass der Benutzer, somit der Tablettenhersteller, nur ein fertiges Stoffgemisch aus einzelnen Tablettierhilfsstoffen mit dem Wirkstoff der Tablette zu mischen braucht. Es entfällt somit das Zudosieren einzelner Tablettierhilfsstoffe, womit Arbeitsaufwand erspart wird und das Problem von Ungenauigkeiten des Dosierens der einzelnen Tablettierhilfsstoffe gar nicht auftritt.

Die Erfindung führt zu den folgenden weiteren Vorteilen:
- Deutlich niedrigere Ausstoßkräfte, dank der gleichmäßigen Verteilung des Schmiermittels in der Tablette und damit eine höhere Tablettiergeschwindigkeit beziehungsweise schnellere Produktion.
- Niedrigere Konzentrationen vom Schmiermittel in der Tablette sind ausreichend.
- Eine bessere Tablettenhärte.
- Einen besseren Abrieb besonders bei niedrigdosierten Wirkstoffen.
- Bessere Fließeigenschaften der Tablettiermasse.
Eine niedrigere Feuchtigkeitsempfindlichkeit.
Keinen Einfluss auf Zerfall und Wirkstofffreigabe.
Kein Overmixing oder Deckeln.
Es ist nur ein Mischschritt mit dem(den) Wirkstoff(en) erforderlich. Das erspart Produktionszeit und Kosten.
Niedrigere Staubzahl.

Das Herstellverfahren der Mischungen aus den oben genannten Komponenten erfolgt durch Wirbelschichtgranulierung.

Im Fall der Wirbelschichtgranulierung gibt es zum Beispiel die folgenden Möglichkeiten:
- **Variante A:** Silicifizierte mikrokristalline Cellulose und Croscarmellose oder Natrium Carboxymethylstärke im Wirbelbett vorlegen und bei 30-97 °C mit einer heißen, wässrigen Lösung von Natriumstearylfumarat besprühen. Das Granulat wird in der Wirbelschicht getrocknet.
- **Variante B:** Mikrokristalline Cellulose und Croscarmellose oder Natrium Carboxymethylstärke in der Wirbelschicht vorlegen und bei 30-97 °C mit einer heißen, wässrigen Lösung/Suspension von Natriumstearylfumarat und Siliciumdioxid besprühen. Das Granulat wird in der Wirbelschicht getrocknet.
- **Variante C:** Mikrokristalline Cellulose, Croscarmellose oder Natrium Carboxymethylstärke und Siliciumdioxid in der Wirbelschicht vorlegen und bei 30-97 °C mit einer heißen, wässrigen Lösung von Natriumstearylfumarat besprühen. Das Granulat wird in der Wirbelschicht getrocknet.
- **Variante D:** Silicifizierte mikrokristalline Cellulose in der Wirbelschicht vorlegen und bei 30-97 °C mit einer heißen, wässrigen Lösung/Suspension von Natriumstearylfumarat und Croscarmellose oder Natrium

Carboxymethylstärke besprühen. Das Granulat wird in der Wirbelschicht getrocknet.
- **Variante E:** Mikrokristalline Cellulose in der Wirbelschicht vorlegen und bei 30-97 °C mit einer heißen, wässrigen Lösung/Suspension von Natriumstearylfumarat, Croscarmellose oder Natrium Carboxymethylstärke und Siliciumdioxid besprühen. Das Granulat wird in der Wirbelschicht getrocknet.

Die Partikelgröße des Wirbelschichtgranulats hängt vom Cellulosetypus ab.

Eine andere nicht erfindungsgemässe Methode zur Herstellung des neuen direktverpressbaren Tablettierhilfsmittels ist die Sprühtrocknung. Alle Komponenten werden in heißem Wasser gemischt und zusammen versprüht. Die Partikelgröße des Produktes hängt vom Cellulosetypus und von der Sprühgeschwindigkeit ab.

Das erfindungsgemäße Tablettierhilfsmittel, das die genannten Komponenten umfasst, nämlich Füller, Bindemittel, Fließregulierungsmittel, Zerfallsbeschleuniger, Schmiermittel, wird durch Granulierung in der Wirbelschicht hergestellt. Siehe die möglichen, nachstehenden Varianten A und B:

### Variante A:

In der Wirbelschicht legt man die silicifizierte mikrokristalline Cellulose (PROSOLV SMCC® 90, JRS Pharma) und Natrium Carboxymethylstärke (EXPLOTAB®, JRS Pharma) vor. Das Material wird auf 30-97 °C erhitzt und mit einer heißen wässrigen Lösung aus 0,3-6 % Natriumstearylfumarat (PRUV®, JRS Pharma) besprüht.
Das Granulat wird in der Wirbelschicht getrocknet.

### Variante B:

In der Wirbelschicht legt man die mikrokristalline Cellulose (VIVAPUR® 102, JRS Pharma) und die Natrium Carboxymethylstärke (EXPLOTAB®, JRS Pharma) vor. Das Material wird auf 30-97 °C erhitzt und mit einer heißen wässrigen Suspension aus 1-20 % Siliciumdioxid (CarbOsil M5®, Cabot oder Aerosil®, Degussa) und 0,3-6 % Natriumstearylfumarat (PRUv®, JRS Pharma) besprüht.
Das Granulat wird in der Wirbelschicht getrocknet.

Die beiden Varianten geben ein Granulat von gleicher Qualität. Tabelle 1 zeigt vier Beilspielgranulate hergestellt mit dem oben genannten Verfahren.

**Tabelle 1. Beispiele von Granulaten**

| **#** | **Granulat 1,** % | **Granulat 2,** % | **Granulat 3,** % | **Granulat 4,** % |
|---|---|---|---|---|
| **VIVAPUR® 102** | 96,5 | 96,0 | 95,0 | 94,0 |
| **CabOsil M5®** | 2,0 | 2,0 | 2,0 | 2,0 |
| **Explotab®** | 1,0 | 1,5 | 2,0 | 3,0 |
| **PRUV®** | 0,5 | 0,5 | 1,0 | 1,0 |

| | | | | |
|---|---|---|---|---|
| Fließwinkel: 25-28 °, Rieselfähigkeit: 0,6-0,9 g/s mit FloDex, 4 mm Ringdurchmesser, Schüttgewicht: 340-400 g/1, Feuchte: 4-6 %. | | | | |

### Beispiele

Das erfindungsgemäße Tablettierhilfsmittel wurde mit der physikalischen Mischung aus den einzelnen Komponenten in einer Placebo, einer 40 %-igen Paracetamol und einer Enalapril Formulierung verglichen. Die Ergebnisse sind in der Tabellen 2, 3 und 4 aufgeführt.

**• Placebo Tabletten**

| 100 % DC Tablettierhilfsmittel* | **gegen** | Physikalische Mischung** | |
|---|---|---|---|
| **(Granulat 1)** | | Mikrokristalline Cellulose 96,5 % | |
| | | | |
| | | Siliciumdioxid | 2% |
| | | Explotab® | 1% |
| | | PRUv® | 0,5% |

| | | | |
|---|---|---|---|
| *Das erfindungsgemäße Tablettierhilfsmittel wird allein bei verschiedenen Presskräften verpresst. **Alle Komponenten ohne PRUV werden für 15 min gemischt. Dann gibt man das Schmiermittel zu, mischt noch 3 min und verpresst bei verschiedenen Presskräften. | | | |

**Tabelle 2: Direkter Vergleich zwischen einem Tablettierhilfsmittel (Granulat 1) und einer physikalischen Mischung aus den einzelnen Komponenten; Placebo Formulierung**

| **Material** | **Presskraft [kN]** | **Bruchfestigkeit [MPa]** | **Ausstoßkraft [N]** | **Abrieb [%]** | **Zerfall [s]** |
|---|---|---|---|---|---|
| **Granulat 1** | 10 | 5,0 | 120 | 0,31 | 10 |
| **Physikalische Mischung** | 10 | 3,9 | 220 | 0,76 | 10 |

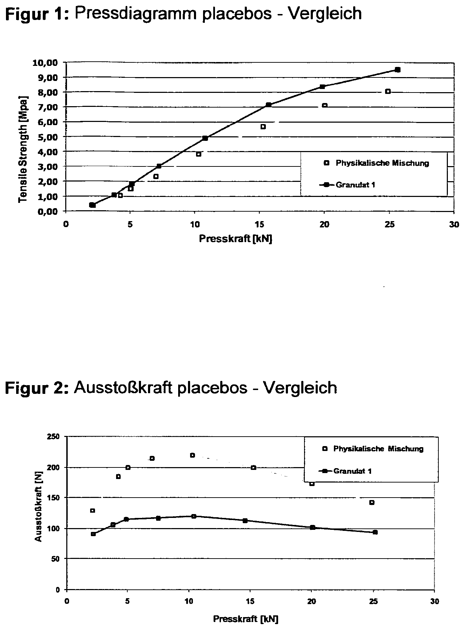

Das erfindungsgemäße DC-Tablettierhilfsmittel zeigt eine deutlich bessere Kompressibilität und Tablettenhärte, niedrigere Ausstoßkraft und geringeren Abrieb als die physikalische Mischung (Tabelle 2, Figur 1 und 2).

**• Paracetamol Tabletten**

| 60 % DC Tablettierhilfsmittel* | **gegenüber** | Physikalische Mischung** | |
|---|---|---|---|
| (**Granulat 1**) | | Mikrokristalline Cellulose 57,9% | |
| 40 % Paracetamol | | Siliciumdioxid | 1,2 % |
| | | Explotab® | 0,6 % |
| | | Paracetamol | 40 % |
| | | PRUV® | 0,3 % |

| | | | |
|---|---|---|---|
| *Das DC Tablettierhilfsmittel wird mit dem Paracetamol 15 min gemischt und dann bei verschiedenen Presskräften verpresst. **Alle Komponenten ohne PRUV werden für 15 min gemischt. Dann gibt man das Schmiermittel zu, mischt noch 3 min und verpresst bei verschiedenen Presskräften. | | | |

**Tabelle 3: Vergleich zwischen der direktverpressbaren Tablettiermischung (Granulat 1) und der physikalischen Mischung aus den einzelnen Komponenten; 40 %-ige Paracetamol (PCM) Formulierung**

| **Material** | **Presskraft [kN]** | **Bruchfestigkeit [MPa]** | **Ausstoßkraft [N])** | **Abrieb [%]** | **Zerfall [s]** |
|---|---|---|---|---|---|
| **Granulat 1** | 15 | 2,2 | 240 | 0,40 | 9 |
| **Physikalische Mischung** | 15 | 1,1 | 380 | 0,51 | 9 |

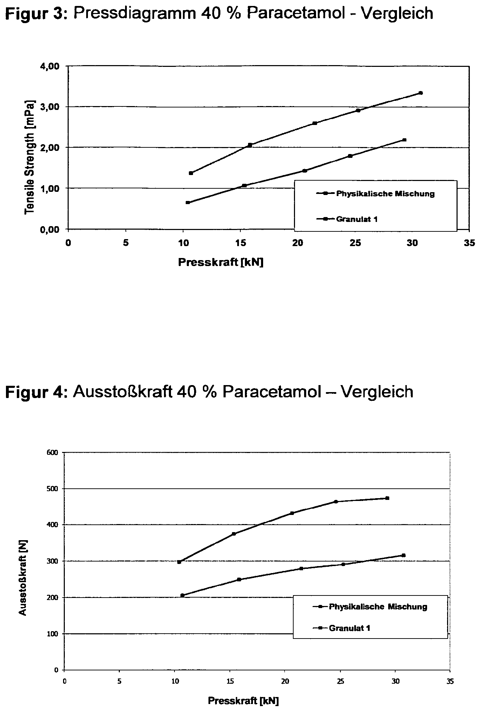

Die PCM Tabletten mit dem neuen DC Tablettierhilfsmittel zeigen deutlich niedrigere Ausstoßkräfte und besseren Abrieb bei den gleichen Zerfallszeiten. Weiterhin benötigt das neue Material eine 50 % niedrigere Presskraft für die gleiche Tablettenhärte zum Vergleich mit der physikalischen Mischung, was auf eine bessere Verpressbarkeit hinweist (Tabelle 3, Figur 3 und 4).

**• Enalapril Tabletten**

| 92,30 % DC Tablettierhilfsmittel* | **gegen** | Physikalische Mischung** | |
|---|---|---|---|
| (**Granulat 1**) | | Mikrokristalline Cellulose 89,07 % | |
| 7,70 % Enalapril | | Siliciumdioxid | 1,85 % |
| | | Explotab® | 0,92 % |
| | | Enalapril | 7,70 % |
| | | PRUV® | 0,46 % |

| | | | |
|---|---|---|---|
| * Das DC Tablettierhilfsmittel wird mit dem Enalapril 15 min gemischt und dann bei verschiedenen Presskräften verpresst. **Alle Komponenten ohne PRUV werden 15 min gemischt. Dann gibt man das Schmiermittel zu, mischt noch 3 min und verpresst bei verschiedenen Presskräften. | | | |

**Tabelle 4: Direkter Vergleich vom direktverpressbaren Tablettierhilfsmittel (Granulat 1) gegen die physikalische Mischung aus den einzelnen Komponenten; Enalapril Formulierung**

| **Material** | **Presskraft [kN]** | **Bruchfestigkeit [MPa]** | **Ausstoßkraft [N]** | **Abrieb [%]** | **Zerfall [s]** |
|---|---|---|---|---|---|
| **Granulat 1** | 15 | 4,0 | 180 | 0,04 | 19 |
| **Physikalische Mischung** | 15 | 1,6 | 260 | 0,04 | 26 |

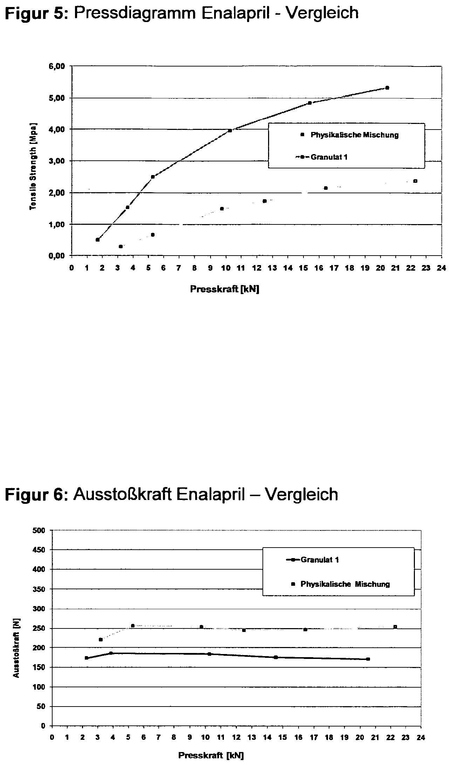

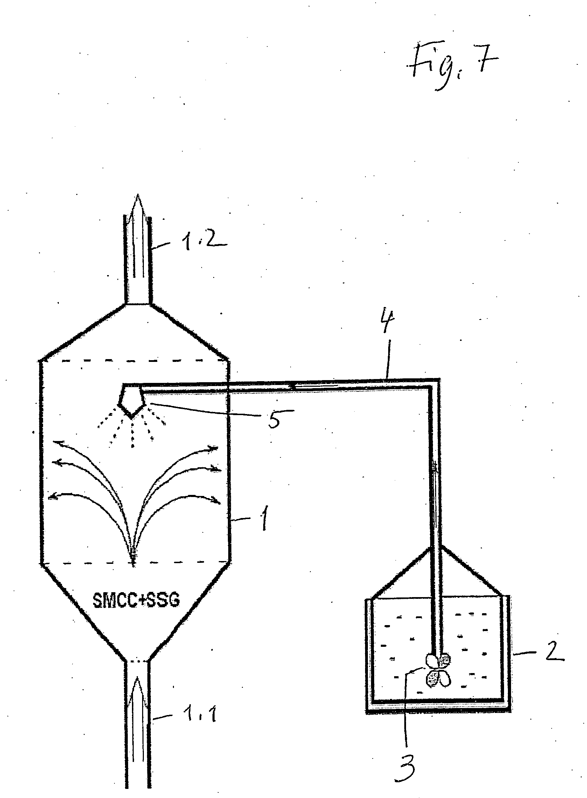

Die Granulat-Formulierung zeigt eine deutlich bessere Verpressbarkeit als die physikalische Mischung (Tabelle 4, Figur 5 und 6). Bei der gleichen Presskraft gibt das neue Tablettierhilfsmittel mehr als doppelte Tablettenhärte (Figur 5) und zeigt eine um 30 % niedrigere Ausstoßkraft.

### Figur 7:

Eine Vorrichtung zum Durchführen des erfindungsgemäßen Verfahrens ist in der Figur dargestellt. Die Vorrichtung umfasst die folgenden Bauteile:
Ein Behälter 1 dient der Aufnahme von SSG (Sodiumstärkeglycolat) und SMCC. Aus diesen beiden Komponenten wird eine Wirbelschicht gebildet. Siehe den Lufteinlass 1.1 und den Luftauslass 1.2. In einem zweiten Behälter 2 befindet sich ein Schmiermittel in flüssiger Form, beispielsweise NSF (Natriumstearylfumarat). Dieses wird durch einen Rührer 3 in ständiger Bewegung gehalten und zugleich über eine nicht dargestellte Pumpe durch eine Rohrleitung 4 zu einer Düse 5 gefördert. Aus der Düse 5 wird es auf die Wirbelschicht aufgesprüht. Das Endprodukt ist das erfindungsgemäße . Tablettierhilfsmittel. Dieses kann vom Tablettenherst ller direkt verwendet werden, indem es mit dem Tablettenwirkstoff gemischt wird. Es bedarf somit nur eines einzigen Zudosiervorganges. Auch kann das erfindungsgemäße Tablettierhilfsmittel gelagert und für beliebige Prozesszeitpunkte bereitgestellt werden

### Bezugszeichenliste

- 1: Behälter zum Herstellen einer Wirbelschicht
- 1.1: Lufteinlass
- 1.2: Luftauslass
- 2: Behälter zur Aufnahme eines Schmiermittels
- 3: Rührer
- 4: Rohrleitung
- 5: Sprühdüse

## Patentansprüche

1. Verfahren zum Herstellen eines Tablettierhilfsmittels bestehend aus den folgenden Verfahrensschritten:
es werden die folgenden Komponenten bereitgestellt:
59-98% eines Füllstoffs/Bindemittels, ausgewählt aus einem oder mehreren der folgenden Komponenten: Cellulose ausgewählt aus mikrokristalliner Cellulose und Pulvercellulose; Cellulose Derivate ausgewählt aus Methyl- und Ethylcellulose, Hypromellose und Hydroxypropylcellulose; Saccharide ausgewählt aus Laktose, Glucose, Saccharose, Fruktose und Kombination von diesen; und Polyole ausgewählt aus Mannitol, Sorbitol, Xylitol, Isomalt und Kombination von diesen;
0,5-15% eines Zerfallsbeschleunigers, ausgewählt aus einem oder mehreren der folgenden Komponenten:
Croscarmellose Natrium, Stärke, Natrium Carboxymethylstärke, quervernetztes Polyvinylpyrrolidon, Soja-Polysaccharid, Cyclodextrin, Xylan, Pektine, Gelatine, Polymethacrylsäure, lonenaustauscherharze;
optional 1-20% eines Fließregulierungsmittels, ausgewählt aus einem oder mehreren der folgenden Komponenten: Siliciumdioxid, Calcium-, Magnesium- und Aluminiumsilikate;
Vermischung und Erwärmung der bereitgestellten Komponenten auf 30 bis 97° C und Besprühung mit einer heißen wässrigen Lösung/Suspension aus 0,3 bis 6% Natriumstearylfumarat, wobei der Zerfallsbeschleuniger und das Fließmittel entweder zusammen mit dem Füllstoff/Bindemittel in der Wirbelschicht vermischt werden oder in der heißen wässrigen Lösung/Suspension aus 0,3 bis 6 % Natriumstearylfumarat enthalten sind.

2. Tablettierhilfsmittel, hergestellt mittels eines Verfahrens gemäss Anspruch 1, umfassend:
59-98% eines Füllstoffs/Bindemittels, umfasst eine oder mehrere der folgenden Komponenten:
Cellulose ausgewählt aus mikrokristalliner Cellulose und Pulvercellulose; Cellulose Derivate ausgewählt aus Methyl- und Ethylcellulose, Hypromellose und Hydroxypropylcellulose; Saccharide ausgewählt aus Laktose, Glucose, Saccharose, Fruktose und Kombination von diesen; und Polyole ausgewählt aus Mannitol, Sorbitol, Xylitol, Isomalt und Kombination von diesen;
1-20% eines Fließregulierungsmittels, das ein oder mehrere der folgenden Komponenten aufweist:
Siliciumdioxid, Calcium-, Magnesium- und Aluminiumsilikate;
0,5-15% eines Zerfallsbeschleunigers, umfasst eine oder mehrere der folgenden Komponenten:
Croscarmellose Natrium, Stärke, Natrium Carboxymethylstärke, quervernetztes Polyvinylpyrrolidon, Soja-Polysaccharid, Cyclodextrin, Xylan, Pektine, Gelatine, Polymethacrylsäure, lonenaustauscherharze;
0,3-6% eines Schmiermittels, das Natriumstearylfumarat umfasst.

## Claims

1. Method for producing a tabletting aid, consisting of the following method steps:
the following components are provided:
59-98% of a filler/binder, selected from one or more of the following components:
cellulose selected from microcrystalline cellulose and powdered cellulose; cellulose derivatives selected from methyl and ethyl cellulose, hypromellose and hydroxypropyl cellulose; saccharides selected from lactose, glucose, sucrose, fructose and combinations thereof; and polyols selected from mannitol, sorbitol, xylitol, isomalt and combinations thereof;
0.5-15% of a disintegrant, selected from one or more of the following components:
croscarmellose sodium, starch, sodium carboxymethyl starch, crosslinked polyvinylpyrrolidone, soy polysaccharide, cyclodextrin, xylan, pectins, gelatins, polymethacrylic acid, ion exchange resins;
optionally 1-20% of a flow regulating agent, selected from one or more of the following components: silicon dioxide, calcium silicates, magnesium silicates and aluminium silicates;
mixing and heating the provided components to 30 to 97°C and spraying with a hot aqueous solution/suspension of 0.3 to 6% sodium stearyl fumarate, wherein the disintegrant and the flow agent either are mixed together with the filler/binder in the fluidized bed or are contained in the hot aqueous solution/suspension of 0.3 to 6% sodium stearyl fumarate.

2. Tabletting aid, produced by means of a method according to claim 1, comprising:
59-98% of a filler/binder, comprises one or more of the following components:
cellulose selected from microcrystalline cellulose and powdered cellulose; cellulose derivatives selected from methyl and ethyl cellulose, hypromellose and hydroxypropyl cellulose; saccharides selected from lactose, glucose, sucrose, fructose and combinations thereof; and polyols selected from mannitol, sorbitol, xylitol, isomalt and combinations thereof;
1-20% of a flow regulating agent, which comprises one or more of the following components:
silicon dioxide, calcium silicates, magnesium silicates and aluminium silicates;
0.5-15% of a disintegrant, comprises one or more of the following components:
croscarmellose sodium, starch, sodium carboxymethyl starch, crosslinked polyvinylpyrrolidone, soy polysaccharide, cyclodextrin, xylan, pectins, gelatins, polymethacrylic acid, ion exchange resins;
0.3-6% of a lubricant, which comprises sodium stearyl fumarate.

## Revendications

1. Procédé pour la fabrication d'un adjuvant pour la fabrication de comprimés, comprenant les étapes suivantes :
apport des composants suivants :
59 à 98 % d'un agent de charge/liant choisi parmi un ou plusieurs des composants suivants :
cellulose choisie parmi la cellulose microcristalline et la cellulose en poudre, dérivés de cellulose choisis parmi la méthylcellulose et l'éthylcellulose, l'hypromellose et l'hydroxypropylcellulose, saccharides choisis parmi le lactose, le glucose, le saccharose, le fructose et des combinaisons de ceux-ci et polyols choisis parmi le mannitol, le sorbitol, le xylitol, l'isomalt et des combinaisons de ceux-ci ;
0,5 à 15 % d'un accélérateur de désagrégation choisi parmi un ou plusieurs des composants suivants :
croscarmellose sodique, amidon, carboxyméthylamidon sodique, polyvinylpyrrolidone réticulée, polysaccharide de soja, cyclodextrine, xylane, pectine, gélatine, acide polyméthacrylique, résines échangeuses d'ions ;
facultativement 1 à 20 % d'un régulateur de fluidité choisi parmi un ou plusieurs des composants suivants :
dioxyde de silicium, silicates de calcium, de magnésium et d'aluminium ;
mélange et chauffage des composants apportés entre 30 et 97 °C et pulvérisation avec une solution/suspension aqueuse chaude de 0,3 à 6 % de fumarate de stéaryle sodique, l'accélérateur de désagrégation et le fluidifiant étant soit mélangés dans la couche fluidisée en même temps que l'agent de charge/liant, soit contenus dans la solution/suspension aqueuse chaude de 0,3 à 6 % de fumarate de stéaryle sodique.

2. Adjuvant pour la fabrication de comprimés fabriqué selon un procédé selon la revendication 1, contenant :
59 à 98 % d'un agent de charge/liant choisi parmi un ou plusieurs des composés suivantes :
cellulose choisie parmi la cellulose microcristalline et la cellulose en poudre, dérivés de cellulose choisis parmi la méthylcellulose et l'éthylcellulose, l'hypromellose et l'hydroxypropylcellulose, saccharides choisis parmi le lactose, le glucose, le saccharose, le fructose et des combinaisons de ceux-ci et polyols choisis parmi le mannitol, le sorbitol, le xylitol, l'isomalt et des combinaisons de ceux-ci ;
1 à 20 % d'un régulateur de fluidité choisi parmi un ou plusieurs des composants suivants :
dioxyde de silicium, silicates de calcium, de magnésium et d'aluminium ;
0,5 à 15 % d'un accélérateur de désagrégation choisi parmi un ou plusieurs des composants suivants :
croscarmellose sodique, amidon, carboxyméthylamidon sodique, polyvinylpyrrolidone réticulée, polysaccharide de soja, cyclodextrine, xylane, pectine, gélatine, acide polyméthacrylique, résines échangeuses d'ions ;
0,3 à 6 % d'un lubrifiant contenant du fumarate de stéaryle sodique.
